# EUROPÄISCHE PATENTSCHRIFT

(11) **EP 2 504 062 B1**
(45) Veröffentlichungstag und Bekanntmachung des Hinweises auf die Patenterteilung: **31.07.2019**
(21) Anmeldenummer: 10779720.1
(22) Anmeldetag: 16.11.2010
(51) Int. Cl.: A61N 5/10, G21F 3/00

(54) **SYSTEM ZUM AUFTRAGEN EINER RADIOAKTIVEN SUBSTANZ AUF EIN BIOLOGISCHES GEWEBE**
SYSTEM FOR APPLYING A RADIOACTIVE SUBSTANCE TO A BIOLOGICAL TISSUE
SYSTÈME POUR APPLIQUER UNE SUBSTANCE RADIOACTIVE SUR UN TISSU BIOLOGIQUE

(30) Priorität: 24.11.2009 DE 102009054388
(43) Veröffentlichungstag der Anmeldung: 03.10.2012
(73) Patentinhaber: OncoBeta International GmbH, 88214 Ravensburg (DE)
(72) Erfinder: CIPRIANI, Cesidio, I-00185 Roma (IT); DESANTIS, Maria, I-00192 Roma (IT); BICHLMAIER, Max, 85664 Hohenlinden (DE); FÖRG, Siegfried, 85669 Pastetten (DE); TUOMO, Nikula, 85521 Ottobrunn (DE); HARFENSTELLER, Mark, 85716 Unterschleissheim (DE); BUCK, Oliver, 83457 Bayerisch Gmain (DE)
(74) Vertreter: Hartig, Michael
(86) Internationale Anmeldenummer: PCT/EP2010/006971
(87) Internationale Veröffentlichungsnummer: WO 2011/063903

(56) Entgegenhaltungen:
- WO-A1-97/26037
- DE-A1- 2 626 811
- KR-A- 20090 076 071
- US-A- 2 932 974
- US-A- 4 092 546
- US-A- 4 307 713
- US-A- 5 505 712
- US-A- 5 514 071
- US-A- 5 927 351
- US-A- 6 033 377

## Beschreibung

Die vorliegende Erfindung betrifft einen Applikator zum Auftragen einer radioaktiven Substanz auf ein biologisches Gewebe, insbesondere die menschliche Haut, zu medizinischen Zwecken sowie einen dafür geeigneten Griff und ein entsprechendes Applikationssystem.

In der Nuklearmedizin werden radioaktive Substanzen zur Behandlung von oberflächennahen Gewebepartien, wie beispielsweise der Haut verwendet. Durch die radioaktive Substanz, die häufig als Bestandteil einer Creme oder eines Lacks auf die Haut aufgetragen wird, werden in Abhängigkeit von der Eindringtiefe und der Einwirkzeit der radioaktiven Strahlung oberflächennahe Zellen zerstört. Diese Methode kann beispielsweise zur Therapie von Basalzellenkarzinomen verwendet werden und hat zu erfolgreichen Behandlungen geführt.

Das Aufbringen der radioaktiven Substanz auf die zu behandelnden Hautpartien erfolgte bisher in flüssiger Form, als in einer Creme oder einem Lack enthaltenes Stoffgemisch oder als in einem Pflaster enthaltener radioaktiver Stoff.

Problematisch ist dabei, dass die Behandlung der oberflächennahen Gewebepartien ohne geeignete Hilfsmittel erfolgt, so dass das medizinische Personal zumindest beim Auftragen auf und Entfernen der radioaktiven Substanz von der Hautoberfläche einer hohen Strahlendosis ausgesetzt ist. Darüber hinaus werden Personen auch bei der Vorbereitung der die radioaktive Substanz enthaltenden Cremes, Lacke oder Pflaster mit einer hohen Strahlendosis belastet.

US 4 092 546 A offenbart eine Abschirmvorrichtung zur Aufnahme und zum Halten einer Phiole, die ein zu ladendes Material enthält, an einem Ende und einer hypodermische Spritze mit einer Nadel an ihrem entgegengesetzten Ende. Ein Durchdringen des Septums der Phiole wird durch Bewegen der verschiebbaren Elemente der Abschirmvorrichtung in Richtung der Phiole bewirkt, wobei die Nadel der hypodermischen Spritze das Septum der Phiole durchdringt und in diese eindringt.

US 2 932 974 A offenbart eine Pipette zur Handhabung gefährlicher Flüssigkeiten, die ein gestrecktes Behältnis und ein hohles Element aufweist, das durch eine Wand in der Form eines Kegelstumpfs definiert ist, wobei sich das hohle Element in einer betrieblichen Verbindung mit dem gestreckten Behältnis befindet, wodurch die Flüssigkeit im Behältnis in das hohle Element überfließen kann, wobei die Seitenwand in der Form eines Kegelstumpfs zumindest eine Öffnung aufweist.

US 5 505 712 A offenbart einen Fluid-Applikator mit einem Saugball, einem Schraubdeckel, einer Fingerauflage, einem inneren Stiel und einem äußeren Stiel, der in einem Spatel endet. Der Saugball ist mit dem dünnen röhrenförmigen inneren Stiel verbunden, der sich durch den gesamten Applikator erstreckt und an einer Öffnung im Spatel endet.

US 6 033 377 A offenbart eine Ampulle mit einer durchstechbaren Dichtung, mit der ein Ende der Ampulle verschlossen ist, und einem Kolben, der das andere Ende der Ampulle verschließt. Darüber hinaus umfasst die Ampulle ein Mischelement, das ein Metall enthält und in einem Raum, der für die Aufnahme eines Medikaments vorgesehen ist, bewegbar ist.

KR 2009 007 6071 A offenbar eine Übertragungsvorrichtung für eine hochradioaktive Probe, mit der die radioaktive Strahlenbelastung eines Arbeiters minimiert werden kann.

US 5 927 351 A offenbart ein verbessertes System zur Handhabung von radioaktivem Material zur Verwendung in Spritzen in der Arzneimittelindustrie. Das System umfasst eine Aufziehstation, eine Spritzenabschirmung und zwei pharmazeutische Behälter. Die Aufziehstation umfasst eine Basis mit einem Ständer und zwei daran montierten Armen, um einen ersten radiopharmazeutischen Behälter zu halten, der einen Behälter für radioaktives Material umschließt.

US 4 307 713 A offenbart eine Abschirmung für eine Spritze beim Aufziehen eines radioaktiven Materials aus einer abgeschirmten Phiole.

Die Aufgabe der vorliegenden Erfindung besteht darin, eine Vorrichtung, mit der eine radioaktive Substanz zur medizinischen Behandlung von biologischem Gewebe, insbesondere der Haut, auf diese mit reduzierter Strahlenbelastung für die Behandlung durchführende Personen aufgetragen werden kann, sowie ein entsprechendes System bereitzustellen.

Diese Aufgabe wird gelöst durch ein System umfassend einen Applikator zum Auftragen einer radioaktiven Substanz auf ein biologisches Gewebe mit den Merkmalen gemäß Anspruch 1. Der Applikator zum Auftragen einer radioaktiven Substanz auf ein biologisches Gewebe umfasst einen Behälter zur Aufnahme der radioaktiven Substanz, eine mit dem Behälter verbindbare Applikationseinrichtung, mit der die Substanzverbindung auf das Gewebe aufgetragen werden kann, und zumindest einen Teil einer Fördereinrichtung, mit der die radioaktive Substanz vom Behälter in die Applikationseinrichtung zugeführt werden kann, wenn die Applikationseinrichtung mit dem Behälter verbunden ist.

Mit dem Applikator ist es möglich, eine zuvor vorbereitete und abgemessene Menge eines Stoffgemischs, das eine radioaktive Substanz zur Behandlung von biologischem Gewebe enthält, im Zeitraum vor der Behandlung im Behälter des Applikators zu transportieren und zu lagern und mit Hilfe der Applikationseinrichtung im Applikator unmittelbar auf ein biologisches Gewebe, wie beispielsweise die menschliche Haut, aufzutragen. Da das im Behälter enthaltene Stoffgemisch bereits vor der Anwendung gegebenenfalls automatisiert hergestellt und hinsichtlich der gewünschten Strahlungsdosis gemessen werden kann, ist die Behandlung einfach durchführbar, ohne dass zusätzliche vorbereitende Schritte durchgeführt werden müssen. Unmittelbar vor dem Auftragen wird lediglich die Applikationseinrichtung mit dem Behälter des für einen Patienten vorbereiteten Applikators verbunden. Mit Hilfe der Fördereinrichtung kann eine präzise Dosierung des Stoffgemisches in die Applikationseinrichtung und auf das Gewebe erfolgen. Bei der Fördereinrichtung kann es sich beispielsweise um einen Kolben oder Stempel handeln, der gegenüber den Wänden des Behälters abgedichtet ist und manuell oder mit Hilfe eines Antriebs in den Behälter gedrückt wird. Die Fördereinrichtung kann als ein manuell bedienbarer Kolben oder Stempel ausgeführt sein.

Ein weiterer Vorteil des Applikators besteht darin, dass der Applikator als für eine individuelle Behandlung bereitgestellter Einwegartikel ausgebildet sein kann, wobei in dem Behälter eine individuelle Menge eines Stoffgemisches für einen Patienten bereitgestellt wird und der Applikator nach der Behandlung vollständig entsorgt wird, so dass eine Kontaminierung von Personen oder Reinigungseinrichtungen vermieden werden kann.

Der Behälter weist eine Öffnung zur Verbindung der Applikationseinrichtung mit dem Behälter auf und einen Verschluss, mit dem die Öffnung verschlossen werden kann. Mit Hilfe des Verschlusses kann der Behälter vor einer Verwendung von der Applikationseinrichtung getrennt werden und der Austritt der radioaktiven Substanz in die Applikationseinrichtung vermieden werden. Zur besseren Abschirmung der Umgebung vor vom Wirkstoff ausgehender radioaktiver Strahlung ist es denkbar, den Behälter aus einem abschirmenden Material, wie beispielsweise Wolfram herzustellen oder ihn mit einer abschirmenden gegebenenfalls abnehmbaren Hülle oder Ummantelung zu umgeben.

Der Applikator umfasst einen Lagerungszustand, in dem der Behälter und die Applikationseinrichtung aneinander befestigt sind, wobei jedoch ein Zuführen des Wirkstoffes oder der radioaktiven Substanz vom Behälter in die Applikationseinrichtung durch den zwischen der Applikationseinrichtung und dem Behälter angeordneten Verschluss verhindert ist. Der Applikator kann gemäß dieser Ausführungsform als eine zur einmaligen Verwendung vorgesehene patienten- oder behandlungsspezifizierte Vorrichtung bereitgestellt werden und durch Verbinden der Applikationseinrichtung und des Behälters durch Beseitigen des Verschlusses einsatzbereit gemacht werden. Bei dem Verschluss kann es sich beispielsweise um eine Dichtung, insbesondere ein Siegel, ein Septum oder einen sonstigen Verschluss handeln, die durchbrochen werden können, indem die Applikationseinrichtung in den Behälter gedrückt wird.

Weiterhin umfasst die Applikationseinrichtung eine Leitung, die zur Herstellung der Verbindung in den Behälter eingeführt werden kann, um die radioaktive Substanz nach der Herstellung der Verbindung zur Applikationseinrichtung zuführen zu können. Bei der Leitung kann es sich beispielsweise um eine Kapillare, eine Nadel oder ein Röhrchen handeln, die durch den Verschluss in den Behälter gedrückt oder geschoben werden kann. Alternativ ist es auch denkbar, den Verschluss vor dem Einführen der Leitung in den Behälter zu entfernen.

Es handelt sich bei der Applikationseinrichtung um einen Pinsel, einen Spatel, einen Schwamm, ein Röhrchen, ein Sieb oder eine Nadel, mit dem bzw. der die radioaktive Substanz auf das biologische Gewebe aufgetragen werden kann. Die Eignung einer oder mehrerer der genannten Applikationseinrichtungen zum Auftragen eines bestimmten eine radioaktive Substanz enthaltenden Stoffgemischs kann von der verwendeten radioaktiven Substanz, ihrer Viskosität, der zur Behandlung vorgesehenen Radioaktivität oder der Art des zu behandelnden Gewebes abhängen.

Gemäß einer bevorzugten Ausführungsform umfassen der Applikator und insbesondere der Behälter zumindest einen Teil einer Mischvorrichtung. Bei der Mischvorrichtung kann es sich beispielsweise um eine magnetische Mischvorrichtung handeln, die einen in dem Behälter angeordneten Mischkörper umfasst, der mittels magnetischer Kopplung an eine außerhalb des Behälters oder des Applikators angeordnete Misch- oder Rühreinrichtung gekoppelt werden kann. Bei der im Behälter enthaltenen Mischeinrichtung kann es sich beispielsweise um eines oder mehrere magnetisierbare Kügelchen, Rotoren oder anders geformte Teile handeln, die mit Hilfe der magnetischen Kopplung mit der Mischeinrichtung durch den Behälter bewegt werden.

Der Behälter weist die Form eines Röhrchens auf. Die Applikationseinrichtung ist an einem Ende des Röhrchens angeordnet und befestigt und bildet eine Fortsetzung davon. Der Applikator weist somit insgesamt eine gestreckte Form oder Stabform auf. Die Applikationseinrichtung kann direkt am Röhrchen befestigt sein. Alternativ kann auch ein Verbindungsstück zwischen der Applikationseinrichtung und dem Behälter verwendet werden. Wenn der Behälter die Form eines Röhrchens aufweist, kann die darin enthaltene Mischeinrichtung die Form eines metallischen, magnetisierbaren zylindrisch geformten Teils aufweisen, dessen Außendurchmesser im Wesentlichen dem Innendurchmesser des Röhrchens entspricht.

Zusätzlich kann die Applikationseinrichtung mit einer Kappe versehen sein. Die Applikationseinrichtung kann darüber hinaus an ihrem dem Röhrchen zugewandten Ende einen röhrenförmigen Abschnitt aufweisen, der formschlüssig in einem Abschnitt des Röhrchens oder des Verbindungsstücks verschiebbar gehalten ist. Damit kann die Applikationseinrichtung in das Röhrchen hineingedrückt werden, wobei der zwischen der Applikationseinrichtung und dem Röhrchen angeordnete Verschluss durchbrochen und eine Verbindung zwischen der Applikationseinrichtung und dem Röhrchen hergestellt wird. Alternativ kann die Applikationseinrichtung auch an einem Verbindungsstück beweglich angeordnet sein, wobei das Verbindungsstück seinerseits auf dem Röhrchen befestigt ist und den Verschluss umfasst.

Darüber hinaus kann der Applikator so ausgebildet sein, dass sich die Applikationseinrichtung im Lagerungszustand, d.h. im vom Behälter getrennten Zustand, in einer ersten Raststellung befindet und beim Hineindrücken in das Röhrchen in eine zweite Raststellung gelangt. Der Applikator kann ferner so aufgebaut sein, dass die Applikationseinrichtung, nachdem sie in den Behälter eingeführt wurde, nicht wieder in den ursprünglichen Lagerungszustand zurückgeführt werden kann, um einen Austritt des die radioaktive Substanz enthaltenden Stoffgemisches zu vermeiden.

Erfindungsgemäß wird ein System umfassend einen Applikator und einen Griff zum Halten des Applikators bereitgestellt. Der Griff umfasst eine Aufnahmevorrichtung für den Applikator, zumindest eine mit der Fördereinrichtung des Applikators koppelbare Antriebsvorrichtung zum Zuführen der radioaktiven Substanz in die Applikationseinrichtung und eine Abschirmungseinrichtung für radioaktive Strahlung, insbesondere zum Schutz der Hand, mit der der Griff gehalten wird.

Der Griff kann mit einer Vielzahl von baugleichen oder ähnlichen Applikatoren zur individuellen Behandlung von Patienten wieder verwendet werden. Durch Verwendung des Griffs mit der daran angeordneten Abschirmungseinrichtung kann eine die Behandlung durchführende Person wirksam vor der radioaktiven Strahlung der zur Behandlung verwendeten radioaktiven Substanz geschützt werden. Darüber hinaus wird eine präzise Dosierung bzw. Zuführung der radioaktiven Substanz auf ein zu behandelndes biologisches Gewebe ermöglicht.

Gemäß einer bevorzugten Ausführungsform wird die Aufnahmevorrichtung durch eine Spanneinrichtung gebildet, mit welcher der Applikator lösbar gehalten werden kann. Vorzugsweise wird der Applikator so in den Griff eingeführt, dass der Behälter des Applikators von der Spanneinrichtung des Griffs gehalten oder umfasst wird, während die Applikationseinrichtung des Applikators vom Griff nach vorne weggerichtet ist. Mit Hilfe des Griffs können Applikatoren mit der durch die Abschirmungseinrichtung des Griffs geschützten Hand ergriffen und nach einer erfolgen Behandlung vom Griff gelöst werden, ohne dass ein Ergreifen des Applikators mit der Hand erforderlich ist.

Gemäß einer weiteren bevorzugten Ausführungsform umfasst die Antriebsvorrichtung des Griffs eine Vorschubmechanik, mit der eine Schubkraft auf die Fördereinrichtung des Applikators ausgeübt werden kann. Durch die Antriebsvorrichtung und die Vorschubmechanik kann die im Behälter des Applikators enthaltene radioaktive Substanz in die Applikationseinrichtung zugeführt werden und bei präziser Dosierung auf das zu behandelnde Gewebe aufgetragen werden. Bei der Fördereinrichtung handelt es sich um einen Kolben oder Stempel, der im Behälter aufgenommen ist. Die Vorschubmechanik ist vorzugsweise so am Griff angeordnet, das ein in der Spannvorrichtung gehaltener Applikator so gehalten ist, dass eine Kopplung zwischen der Fördereinrichtung desselben und der Vorschubmechanik erfolgen kann.

Gemäß einer weiteren bevorzugten Ausführungsform umfasst das System ein getrenntes Handgerät, an dem die Vorschubmechanik angeordnet ist und einen am Griff angeordneten, mit der Fördereinrichtung des Applikators koppelbaren Führungsdraht, der mit der Vorschubmechanik verbunden ist. Gemäß dieser Ausführungsform erfolgt die Durchführung der Behandlung mit Hilfe einer zweiteiligen Vorrichtung, wobei die Dosierung der in die Applikationseinrichtung zugeführten Menge mit dem Handgerät mit der einen Hand erfolgt, während der Griff mit dem Applikator mit der anderen Hand gehalten wird.

Gemäß einer weiteren bevorzugten Ausführungsform umfasst der Griff eine gestreckte Form, wobei an einem Ende die Aufnahmevorrichtung für den Applikator angeordnet ist und der Griff im Inneren einen Kanal zur Aufnahme des Führungsdrahtes aufweist. Der Kanal durchquert den Griff in dessen Längsrichtung bis zur Aufnahmevorrichtung, so dass das Ende des Führungsdrahtes mit dem Applikator oder mit der Fördereinrichtung desselben gekoppelt werden kann.

Gemäß einer weiteren Ausführungsform weist ein Teil der Aufnahmevorrichtung eine Abschirmung auf, mit der ein Teil eines in der Aufnahmevorrichtung aufgenommenen Applikators abgedeckt ist. Bevorzugterweise wird der Behälter des Applikators, der in der Aufnahmevorrichtung aufgenommen ist und der die radioaktive Substanz enthält, durch eine Abschirmung des Griffs, die die Aufnahmevorrichtung umgibt, abgeschirmt.

In einer Ausführungsform umfasst das System einen Transportbehälter zur Aufbewahrung eines oder mehrerer der erfindungsgemäßen Applikatoren. Gemäß einer bevorzugten Ausführungsform umfasst der Transportbehälter einen Teil einer magnetischen Mischvorrichtung, die mit einem im Applikator angeordneten weiteren Teil der Mischvorrichtung durch magnetische Kopplung gekoppelt werden kann. Darüber hinaus kann der Transportbehälter so ausgestaltet sein, dass jeweils nur ein Applikator daraus entnommen werden kann. Dazu kann eine entsprechende Verriegelungsmechanik am Transportbehälter vorgesehen werden.

Gemäß einer weiteren bevorzugten Ausführungsform umfasst das System ein Gestell, an welchem der Transportbehälter und eine Halterung für den Griff beweglich angeordnet sind, so dass der in der Halterung angeordnete Griff zum Transportbehälter zur Entnahme eines dort aufbewahrten Applikators bewegt werden kann. Dadurch wird eine Aufnahme eines Applikators mit dem Griff ermöglicht, ohne dass der Griff dazu mit der Hand ergriffen werden muss. Dadurch kann die Strahlungsdosis, der Personen, die den Griff betätigen, ausgesetzt werden, weiter reduziert werden. Das Gestell kann fahrbar ausgeführt sein.

In einer Variante umfasst das System eine Messeinheit zum Messen der auf das biologische Gewebe aufgetragenen Radioaktivität und einen abgeschirmten Abfallbehälter. Bei der Messeinheit kann es sich beispielsweise um einen Alpha-, Beta- oder Gammadetektor, wie beispielsweise um einen Szintillationsdetektor oder Kalibrator handeln, mit der die Aktivität der in einem Applikator enthaltenen radioaktive Substanz vor und nach dem Auftragen auf das biologische Gewebe gemessen wird und aus der Differenz der Aktivitäten im Applikator die Aktivität der aufgetragenen radioaktiven Substanz ermittelt wird. Die Aktivität der auf das biologische Gewebe aufgetragenen radioaktiven Substanz kann jedoch auch durch Messen, insbesondere optisches Messen, der Fläche, auf der die radioaktive Substanz auf dem biologischen Gewebe aufgetragen wurde, und der Flächenaktivität, d.h. der Aktivität pro Einheitsfläche, mit einem geeigneten Messkopf erfolgen.

In einer weiteren Variante sind der Abfallbehälter und die Messeinheit beweglich am Gestell angeordnet, so dass der in der Halterung angeordnete Griff zum Abfallbehälter zur Aufnahme eines Applikators bzw. zur Messung der Radioaktivität eines Applikators bewegt werden kann.

In einer weiteren Variante sind der Transportbehälter, die Messeinheit und der Abfallbehälter drehbar, beispielsweise auf einem Drehteller, angeordnet, während die Halterung linear beweglich über dem Drehteller angeordnet ist, so dass ein in der Halterung gehaltener Griff in den Transportbehälter, die Messeinheit und den Abfallbehälter abgesenkt werden kann, um einen Applikator aufzunehmen, die von ihm ausgehende radioaktive Strahlung zu messen oder den Applikator abzulegen.

Weitere Eigenschaften, Merkmale und Vorteile der Erfindung ergeben sich aus der Beschreibung einer beispielhaften Ausführungsform in Verbindung mit der begleitenden Zeichnung, in welcher:
Fig. 1a, b eine schematische Darstellung im Schnitt des erfindungsgemäßen Applikators gemäß einer Ausführungsform in einem Lagerungszustand (Fig. 1a) und in einem Gebrauchszustand (Fig. 1b) zeigen;
Fig. 2 einen erfindungsgemäßen Griff zur Verwendung mit dem erfindungsgemäßen Applikator gemäß der in Fig. 1a, 1b gezeigten Ausführungsform in einer Schnittansicht zeigt;
Fig. 3 den erfindungsgemäßen Griff gemäß der in Fig. 2 gezeigten Ausführungsform und ein damit verwendbares Handgerät gemäß einer bevorzugten Ausführungsform in einer Schnittansicht zeigt;
Fig. 4 einen Transportbehälter zur Aufnahme und Lagerung mehrerer erfindungsgemäßer Applikatoren gemäß der in Fig. 1a, 1b gezeigten Ausführungsform in einer Schnittansicht zeigt;
Fig. 5 ein Applikationssystem gemäß einer Ausführungsform der Erfindung in dreidimensionaler Ansicht zeigt;

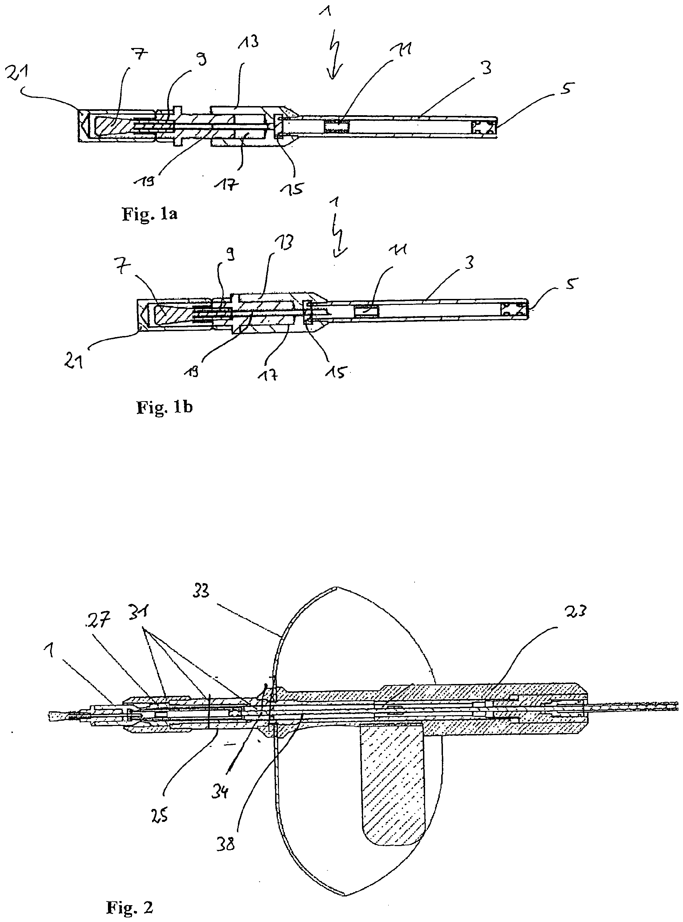

Im Folgenden werden der erfindungsgemäße Applikator 1, der Griff und das Applikationssystem anhand einer beispielhaften Ausführungsform unter Bezugnahme auf die Figuren 1-5 beschrieben. Gemäß der beschriebenen Ausführungsform handelt es sich bei dem Applikator 1 um ein Karpulensystem mit einem als röhrenförmigen Behälter 3 ausgebildeten Grundkörper zur Aufnahme einer Wirkstoffmasse oder Wirkstoffverbindung, die eine radioaktive Substanz für eine medizinische Behandlung eines biologischen Gewebes, wie beispielsweise der Haut enthält. Die Wirkstoffmasse oder Wirkstoffverbindung kann jede für eine Behandlung von Gewebe geeignete Kombination aus einer oder mehreren radioaktiven Substanzen, wie beispielsweise Rhenium, und Zusatzstoffen umfassen. Die Wirkstoffmasse oder Wirkstoffverbindung kann in einer flüssigen Form, als Creme oder Lack oder als Suspension vorliegen.

Der Applikator 1 ist an einer Seite mit einem zylinderförmigen beweglichen, dichtenden Kolben oder Stempel 5, beispielsweise einem zylindrischen massiven oder hohlen Metallteil mit integrierten 0-Ringen, abgedichtet. Der Stempel 5 ist so im Behälter 3 angeordnet, dass durch Ausübung von Druck auf die nach außen orientierte Seite des Stempels 5 die Wirkstoffverbindung aus dem Behälter 3 in eine mit einem Pinsel 7 ausgestattete Applikationseinrichtung 9 des Applikators 1 gedrückt wird. Der Stempel 5 kann schon vor der Befüllung mit der Wirkstoffverbindung im Behälter 3 montiert sein.

Zur Durchmischung der Wirkstoffverbindung ist im Behälter 3 ein Mischeinsatz 11 vorgesehen, der aus einem magnetisierbaren Material hergestellt ist und mittels Magnetkräften, die von einer außerhalb des Grundkörpers angeordneten Misch- oder Rühreinrichtung erzeugt werden, im Grundkörper vor und zurück bewegt werden kann. Der vordere Teil des Behälters 3 wird nach der Befüllung mit der Wirkstoffverbindung mit einem Verbindungsstück 13 verschlossen, das ähnlich wie der Behälter 3 eine Röhrenform mit einem inneren Durchmesser aufweist, der dem äußeren Durchmesser des Behälters 3 entspricht. In das Verbindungsstück 13 ist eine Dichtung 15 integriert, die ein Auslaufen der Wirkstoffverbindung während des Transports und der Lagerung verhindert. Die der als Verschluss dienenden Seite gegenüberliegende Seite des Verbindungsstücks 13 ist als zylinderförmige Aufnahme 17 mit einem inneren Durchmesser derart ausgebildet, dass darin passgenau die Applikationseinrichtung 9 aufgenommen werden kann. Die Aufnahme 17 ist darüber hinaus so ausgeführt, dass die Applikationseinrichtung 9 in einer von zwei Raststellungen gehalten wird. In der ersten, in Fig. 1a gezeigten Raststellung befindet sich die Applikationseinrichtung 9 in der Transport- und Lagerposition, in der eine in den Pinsel 7 der Applikationseinrichtung 9 integrierte Kapillare 19 die Dichtung 15 nicht durchstößt. Wird zur Anwendung der Applikator 1 aktiviert, so wird die Applikationseinrichtung 9 in die in Fig. 1b gezeigte zweite Raststellung gedrückt. Der Applikator 1 ist so ausgebildet, dass in der zweiten Raststellung der Pinsel 7 zur Vermeidung von Kontaminationen nicht mehr in die erste Raststellung zurückbewegt werden kann. In der zweiten Raststellung wird zudem die Dichtung 15 von der Kapillare 19 durchstoßen, so dass der Wirkstoff zur Applikation in den Pinsel 7 zugeführt werden kann.

Der Pinsel 7 dient zum Auftragen und Verteilen der Wirkstoffverbindung auf der Gewebeoberflache. Er ist vorzugsweise als Pinsel mit Borsten oder Haaren ausgeführt, kann aber auch durch eine andere geeignete Einrichtung, wie beispielsweise ein Spatel, Rakel oder ein Siebgitter, hergestellt aus elastischem Kunststoff oder aus einem anderen geeigneten Material ersetzt sein. Der Pinsel 7 wird durch eine Abdeckung 21 geschützt. Diese wird nur zur Anwendung der Wirkstoffverbindung entfernt.

Nach der Anwendung wird die Abdeckung 21 wieder auf den Pinsel 7 gesetzt, um eine Kontamination durch den mit der Wirkstoffverbindung benetzten Pinsel 7 zu verhindern. Der Mischeinsatz 11 ist so ausgeführt, dass ein bestmögliches Entleeren der Wirkstoffverbindung aus dem Applikator 1 beziehungsweise der Karpule ermöglicht wird. Im vorliegenden Beispiel hat der Mischeinsatz 11 eine zylindrische Form mit einer zentralen Bohrung. Der Durchmesser der Bohrung ist auf den Durchmesser der Kapillare 19 abgestimmt, so dass die Kapillare 19, wenn sie, wie in Fig. 1b gezeigt ist, in den Behälter 3 eingeführt ist, das Todvolumen in der Bohrung möglichst vollständig ausfüllt. Zudem ist der Mischeinsatz 11 so ausgeführt, dass ein Verkanten desselben während des Mischens oder des Entleerens verhindert wird. Der Behälter 3, die Applikationseinrichtung 9 und das Verbindungsstück 13 können aus Kunststoff oder Glas oder einer Kombination daraus hergestellt sein.

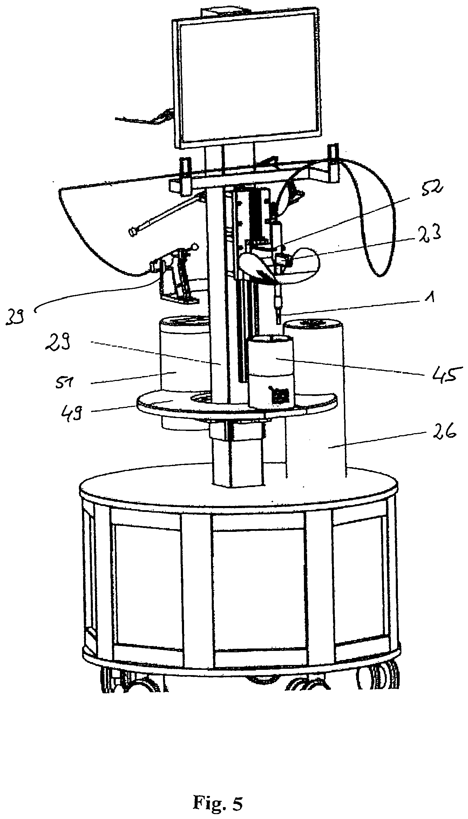

Das System umfasst des weiteren einen speziellen Griff 23, der so ausgebildet ist, dass darin der Applikator 1 aufgenommen werden kann. Mit dem Griff 23 und einem daran gehaltenen Applikator 1 kann der Wirkstoff mit hoher Präzision und genauer Dosierung auf ein biologisches Gewebe aufgebracht werden. Die Spitze 25 des im Allgemeinen Stabförmigen Griffs 23, die zur Aufnahme des Applikators 1 eingerichtet ist, ist so gestaltet, dass sie in ein geeignetes Messsystem, vorzugsweise eine Messstation 26 mit einem Kalibrator zur Bestimmung der Aktivität des Wirkstoffs im Applikator 1 eingeführt werden kann. Der Griff 23 umfasst zum Aufnehmen des Applikators 1 eine Mechanik, die durch einen Spannmechanismus, bevorzugt in Form einer dreiteiligen Spannzange 27, gebildet ist. Die Spannzange 27 wird durch einen Mechanismus in einer speziellen Ladestation 29 entriegelt, die in Fig. 5 gezeigt ist, und kann nur in der Ladestation 29 zum Aufnehmen oder Freigeben des Applikators 1 geöffnet werden. Der vordere Teil des Griffs 21 umfasst eine Abschirmung 31, vorzugsweise aus Wolfram, um von dem im Applikator 1 enthaltenen Wirkstoff ausgehende Strahlung abzuschirmen. Zur Abschirmung der Hand umfasst der Griff 23 zusätzlich einen Schutzschild 33, der beispielsweise aus einer Aluminiumlegierung hergestellt ist. Der Schutzschild 33 hat die Form eines Teilzylinders und ist so ausgeformt, dass das Auftragen der Wirkstoffverbindung nicht beeinträchtigt wird.

Der Wirkstoff wird aus dem Applikator 1 gefördert, indem ein Führungsdraht 37, der in einem den Griff 23 durchquerenden Kanal 38 geführt ist und am Stempel 5 des Applikators 1 aufliegt, den Stempel 5 in Richtung des Pinsels 7 bewegt. Der Führungsdraht 37 wird über ein Handgerät 39 betätigt, das mit einer Hand eines Anwenders gehalten wird, während der Griff 23 mit der anderen gehalten wird. Mittels eines Hebels 41 am Handgerät 39 wird der Führungsdraht 37 durch einen Mechanismus 43 im Handgerät 39 mit in einer geeigneten Untersetzung der Bewegung in Richtung des Applikators 1 gedrückt. Alternativ zum Handgerät 39 kann ein Mechanismus zur Ausführung der Zustellbewegung in den Griff 23 selbst integriert sein, so dass eine Einhandbedienung ermöglicht ist.

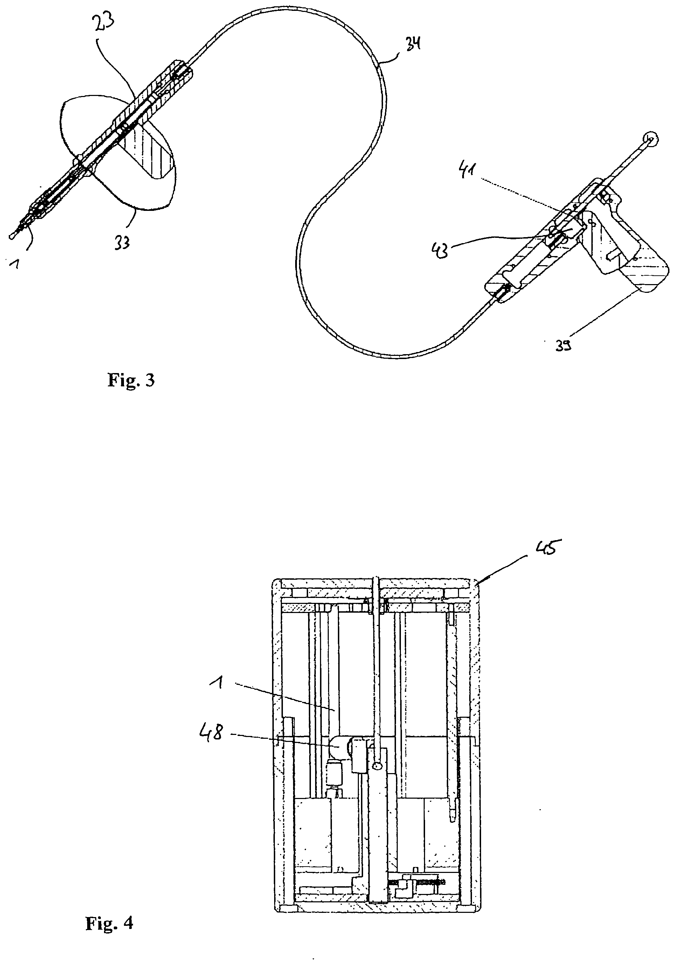

Der in Fig. 4 gezeigte zylinderförmige Transportbehälter 45 dient zur Aufnahme und Lagerung mehrerer, vorzugsweise von sechs Applikatoren 1 bzw. Karpulen, die im Transportbehälter 45 vorzugsweise stehend und in einer kreisförmigen Anordnung aufgenommen und gehalten sind. Eine Verriegelungsmechanik 47 des Transportbehälters 45 stellt aus Strahlenschutzgründen sicher, dass jeweils nur ein Applikator 1 entnommen werden kann. Darüber ist im Transportbehälter 45 ein Mechanismus vorgesehen, der sicherstellt, dass der jeweilige Applikator 1 nur nach einem vorherigen Mischen entnommen werden kann. In den Transportbehälter 45 ist dazu eine Rühreinrichtung mit einem beweglichen Magneten 48 integriert, mit dem der Mischeinsatz 11 des Applikators 1 zum Durchmischen des Wirkstoffes bewegt werden kann.

In Fig. 5 ist ein Beispiel einer Ausführungsform eines fahrbaren Applikationssystems gezeigt. Das Applikationssystem umfasst ein Gestell mit der Ladestation 29, welche die zentrale Einheit des beispielhaft gezeigten Applikationssystems darstellt und an der weitere Komponenten angebracht sind. Das Applikationssystem umfasst des weiteren eine drehbare Einheit, die beispielhaft als Drehteller 49 ausgeführt ist. Auf der drehbaren Einheit sind eine Aufnahme für den Transportbehälter 45, eine Aufnahme für die Messstation 26 und eine Aufnahme für einen Abfalleimer 51 angeordnet. Um eine kollisionsfreie Bewegung des Drehtellers 49 sicherzustellen, befindet sich in geeignetem Abstand über dem Drehteller 49 an der Ladestation 29 eine Halterung 52 für den Griff 23. Die Halterung 52 besitzt einen linearen Freiheitsgrad in vertikaler Richtung, so dass der in der Halterung 52 aufgenommene Griff 23 zum Aufnehmen, Messen und Freigeben des Applikators 1 in den Transportbehälter 45, die Messstation 26 beziehungsweise den Abfalleimer 51 abgesenkt werden kann.

Die Messstation 26 umfasst als Teil eines geeigneten Messsystems einen Kalibrator, der auch Aktivimeter oder Schachtionisationskammer genannt wird, im dem die Spitze des Griffs 23 und der Applikator 1 aufgenommen werden können. Mit Hilfe des Kalibrators wird die Aktivität des Wirkstoffes im Applikator 1 vor und nach der Anwendung bestimmt.

Neben der Aktivitätsmessung mit dem Kalibrator kann die Aktivität eines Wirkstoffs auch direkt auf dem Patienten mit Hilfe eines kollomierten Messkopfes in Verbindung mit einer optischen Messung der Fläche der aufgetragenen Schicht des Wirkstoffes auf dem Patienten gemessen werden. Beide Methoden der Aktivitätsmessung können alternativ oder gemeinsam angewendet werden.

Der Abfalleimer 51 dient zur Aufnahme des verbrauchten Applikators 1 und kann auch zur Aufnahme von Wirkstoff dienen, der nach der Anwendung vom Gewebe entfernt wurde. Der Abfalleimer 51 besitzt eine ausreichende Abschirmung, die vorzugsweise durch eine Schicht aus einem Material mit hohem Wasserstoffgehalt, wie beispielsweise Polymethylmethacrylat, um Beta-Strahlen abzuschirmen, und eine Schicht aus einem Material mit hohen Elektronengehalt, wie beispielsweise Wolfram oder Blei, um Gamma-Strahlen einzufangen und abzuschirmen, gebildet ist. Dabei ist die Schicht aus dem Material mit hohem Wasserstoffgehalt weiter innen liegend und vor der Schicht aus dem Material mit hohen Elektronengehalt angeordnet, um auch die an der Schicht aus dem Material mit hohem Wasserstoffgehalt entstehende Bremsstrahlung von Alpha-Teilchen abzuschirmen. Der Deckel des Abfalleimers 51 lässt sich über eine Mechanik bedienen. Beim Öffnen wird eine Kulisse freigelegt, in die der Griff 23 eingeführt wird, um den verbrauchten Applikator 1 zu entsorgen. Gleichzeitig wird eine weitere Öffnung freigegeben, um den bei der Behandlung verwendeten, gegebenenfalls mit einem Klebeband von der Gewebestelle abgenommenen Wirkstoff aufzunehmen.

Zum beispielhaft dargestellten Applikationssystem gehören neben den beschriebenen Komponenten ferner Werkzeuge zum Entfernen des Wirkstoffs von den behandelten Gewebebestellen. Im folgenden wird beispielhaft der Ablauf eines Prozesses zum Auftragen eines eine radioaktive Substanz enthaltenden Wirkstoffes auf ein biologisches Gewebe, wie beispielsweise die Haut beschrieben. Der beispielhafte Prozess gliedert sich in vier Abschnitte, die die Vorbereitung und das Abpacken oder Abfüllen des Wirkstoffes, das Auftragen des Wirkstoffes, das Einwirken des Wirkstoffes während einer Einwirkzeit und das Entfernen und Entsorgen des Wirkstoffes umfassen. Bei dem Wirkstoffverbindung kann es sich um eine eine radioaktive Substanz enthaltende Creme, Flüssigkeit oder Lack handeln.

Nach der Herstellung des Wirkstoffes und der Abfüllung in den Behälter 3 des Applikators 1 wird der gefüllte Applikator 1 wird in den Transportbehälter 45 eingesetzt. Dazu wird die Mechanik des Transportbehälters 45 entriegelt, um den Transportbehälter 45 mit bis zu sechs Applikatoren 1 beladen zu können. Anschließend wird die Verriegelungsmechanik 47 wieder aktiviert, um ein unbefugtes Entnehmen der Applikatoren 1 zu verhindern. Der Transportbehälter 45 wird in die Ladestation 29 eingesetzt. Die folgenden Prozessschritte sind zwangsgeführt, um die richtige Reihenfolge sicher einzuhalten. Der Griff 23 wird in der Halterung 52 an der Ladestation 29 befestigt, um die Spannzange 27 zu entriegeln. Bevor ein neuer Applikator 1 in den Griff 23 geladen werden kann, muss der Mischmechanismus am Transportbehälter 45 betätigt werden. Nun wird der Griff 23 mit geöffneter Spannzange 27 linear in den Transportbehälter 45 bewegt. In der untersten Stellung wird die Spannzange 27 geschlossen und der Applikator 1 im Griff 23 fixiert. Der Griff 23 wird aus dem Transportbehälter 45 linear nach oben herausgefahren. Die drehbare Einheit 49 an der Ladestation 29 wird so weitergedreht, dass der Griff 23 über der Messstation 26 steht. Nun wird der Griff 23 in derselben Weise in die Messstation 26 gesenkt und die Aktivität in dem aufgenommenen Applikator 1 wird bestimmt. Nach diesem Schritt wird der Applikator 1 aktiviert, indem die Kapillare 19 durch die Dichtung 15 geschoben wird. Dadurch wird der Pinsel die Kapillare 19 von der ersten Raststellung in die zweite Raststellung gebracht. Ebenso wird die Abdeckung 21 des Pinsels 7 entfernt.

Das Auftragen der Wirkstoffverbindung kann im nächsten Arbeitsabschnitt beginnen. Dazu wird durch eine Pumpbewegung, ausgeführt am Handgerät 39, der Stempel 5 in kleinen Schritten durch den Führungsdraht 37 bewegt, so dass die Wirkstoffverbindung durch die Kapillare 19 in den Pinsel 7 fließt. Die Wirkstoffverbindung wird durch den Pinsel 7 auf der Gewebeoberfläche gleichmäßig und dünn verteilt. Nach dem erfolgten Auftragen muss die im Applikator 1 verbliebene Wirkstoffverbindung bestimmt werden. Dazu wird der Griff 23 wieder in die Aufnahme der Ladestation 29 gebracht. Der Griff 23 wird in die Messstation 26 gesenkt, um die Restaktivität zu bestimmen. Dabei wird beim Einfahren in die Messstation 26 eine neue Abdeckung auf den Pinsel 7 gesetzt, um eine Kontamination zu vermeiden. Nach der Messung wird die drehbare Einheit 49 der Ladestation 29 so weiterbewegt, dass der Griff 23 über dem Abfalleimer 51 steht. Durch Hineinbewegen des Griffs 23 in den Abfalleimer 51 kann die Spannzange 27 des Griffs 23 wieder entriegelt werden, um den gebrauchten Applikator 1 in den Abfalleimer 51 zu entsorgen.

Die Wirkstoffverbindung muss eine genau bestimmte Zeit auf dem Gewebe verbleiben, um die errechnete Strahlungsdosis abzugeben. Während dieses Schrittes sind keine Arbeitsvorgänge vorgesehen. Die Wirkstoffverbindung härtet oder trocknet während der Einwirkzeit aus.

Nach Ablauf der Einwirkzeit muss die nun feste Wirkstoffverbindung im letzten Arbeitsschritt entfernt werden. Die kann beispielsweise mit einem Klebeband erfolgen, das an einem geeigneten Werkzeug angebracht ist. Die Gewebeoberfläche wird danach mit einem geeigneten Messgerüst auf Kontamination untersucht. Falls eine Kontamination festgestellt wird, muss der gesamte letzte Arbeitsschritt wiederholt werden.

Am Applikator 1, dem Griff 23 und dem Applikationssystem können zahlreiche Modifizierungen vorgenommen werden, ohne vom Umfang der Erfindung abzuweichen.

### Bezugszeichenliste:

- 1.: Applikator
- 3.: Behälter
- 5.: Stempel
- 7.: Pinsel
- 9.: Applikationseinrichtung
- 11.: Mischeinsatz
- 13.: Verbindungsstück
- 15.: Dichtung
- 17.: Zylinderförmige Aufnahme
- 19.: Kapillare
- 21.: Abdeckung
- 23.: Griff
- 25.: Spitze
- 26.: Messstation
- 27.: Spannzange
- 29.: Ladestation
- 31.: Abschirmung
- 33.: Schutzschild
- 34.: Führungsdraht
- 38.: Kanal
- 39.: Handgerät
- 41.: Hebel
- 43.: Mechanismus
- 45.: Transportbehälter
- 47.: Verriegelungsmechanik
- 48.: Magnet
- 49.: Drehteller
- 51.: Abfalleimer
- 52.: Halterung

## Patentansprüche

1. System umfassend einen Applikator (1) zum Auftragen einer radioaktiven Substanz auf ein Gewebe, wobei der Applikator (1) einen rohrförmigen Behälter (3) zur Aufnahme der radioaktiven Substanz und eine mit einem Ende des rohrförmigen Behälters (3) verbindbare Applikationseinrichtung (9) aufweist, mit der die radioaktive Substanz auf das Gewebe aufgetragen werden kann, wobei die Applikationseinrichtung (9) eine Leitung (19), die zur Herstellung einer Verbindung in den Behälter (3) eingeführt werden kann, um nach der Herstellung der Verbindung die radioaktive Substanz zur Applikationseinrichtung (9) zuzuführen, und einen Pinsel, Spatel, Schwamm, ein Röhrchen, oder eine Nadel zum Auftragen der radioaktiven Substanz auf das biologische Gewebe aufweist und der rohrförmige Behälter (3) einen Stempel oder Kolben als einen Teil einer Fördereinrichtung (5) aufweist, mit der die radioaktive Substanz vom Behälter (3) in die Applikationseinrichtung (9) zugeführt werden kann, und der Applikator (1) einen Lagerungszustand aufweist, in dem der Behälter (3) und die Applikationseinrichtung (9) aneinander befestigt sind, wobei ein Zuführen der radioaktiven Substanz vom Behälter (3) in die Applikationseinrichtung (9) durch einen zwischen der Applikationseinrichtung (9) und dem Behälter (3) angeordneten Verschluss (15) verhindert ist, wobei das System einen Griff (23) mit einer Aufnahmevorrichtung für den Applikator (1), zumindest einem Teil einer mit der Fördereinrichtung (5) des Applikators (1) koppelbaren Antriebsvorrichtung zum Zuführen der radioaktiven Substanz in die Applikationseinrichtung (9) des Applikators (1) und einer Abschirmungseinrichtung (31, 33) für radioaktive Strahlung zum Schutz der den Griff haltenden Hand aufweist.

2. System nach Anspruch 1, **dadurch gekennzeichnet, dass** der Behälter (3) zumindest einen Teil (11) einer Mischvorrichtung umfasst.

3. System nach Anspruch 1 oder 2, **dadurch gekennzeichnet, dass** die Aufnahmevorrichtung durch eine Spanneinrichtung (27) gebildet ist, mit welcher der Applikator (1) lösbar gehalten werden kann.

4. System nach einem der Ansprüche 1 bis 3, **dadurch gekennzeichnet, dass** die Antriebsvorrichtung eine Vorschubmechanik (43) umfasst, mit der eine Schubkraft auf die Fördereinrichtung (5) des Applikators (1) ausgeübt werden kann.

5. System nach Anspruch 4, **dadurch gekennzeichnet, dass** er ein vom Griff getrenntes Handgerät (39), an dem die Vorschubmechanik (43) angeordnet ist, und einen am Griff (23) angeordneten, mit der Fördereinrichtung (5) des Applikators (1) koppelbaren Führungsdraht (34) umfasst, der mit der Vorschubmechanik (43) verbunden ist.

6. System nach Anspruch 5, **dadurch gekennzeichnet, dass** der Griff (23) eine gestreckte Form aufweist, wobei an einem Ende die Aufnahmevorrichtung angeordnet ist und der Griff einen Kanal (38) zur Aufnahme des Führungsdrahtes (34) aufweist, wobei der Kanal (38) den Griff (23) in dessen Längsrichtung bis zur Aufnahmevorrichtung durchquert.

7. System nach einem der Ansprüche 1 bis 6, **dadurch gekennzeichnet, dass** ein Teil der Aufnahmevorrichtung eine Abschirmung (31) aufweist, mit der ein Teil eines in der Aufnahmevorrichtung aufgenommenen Applikators (1) abgedeckt ist.

8. System nach einem der Ansprüche 1 bis 7 mit einem Transportbehälter (45) zur Aufbewahrung eines oder mehrerer Applikatoren (1).

9. System nach Anspruch 8, **dadurch gekennzeichnet, dass** der Transportbehälter (45) einen Teil (48) einer magnetischen Mischvorrichtung umfasst, die mit einem im Applikator (1) angeordneten weiteren Teil (11) der Mischvorrichtung durch magnetische Kopplung gekoppelt werden kann.

10. System nach Anspruch 8 oder 9, **dadurch gekennzeichnet, dass** es ein Gestell umfasst, an welchem der Transportbehälter (45) und eine Halterung (52) für den Griff (23) beweglich angeordnet sind, so dass der in der Halterung (52) angeordnete Griff (23) zum Transportbehälter (45) zur Entnahme eines dort aufbewahrten Applikators (1) bewegt werden kann.

## Claims

1. System comprising an applicator (1) for applying a radioactive substance onto a tissue, wherein the applicator (1) comprises a tubular container (3) for receiving the radioactive substance, and an application device (9) which can be connected to one end of the tubular container (3), with which the radioactive substance can be applied onto the tissue, wherein the application device (9) comprises a line (19), which can be introduced into the container (3) in order to establish a connection, in order, after the establishment of the connection, to convey the radioactive substance to the application device (9), and a brush, spatula, sponge, tube, or a needle to apply the radioactive substance onto the biological tissue, and the tubular container (3) comprises a plunger or piston as a part of a conveyor device (5) with which the radioactive substance can be conveyed from the container (3) into the application device (9), and the applicator (1) exhibits a storage state in which the container (3) and the application device (9) are secured to each other, wherein a conveying of the radioactive substance from the container (3) into the application device (9) is prevented by a closure element (15) arranged between the application device (9) and the container (3), and wherein the system comprises a handle (23) with an receiver device for the applicator (1), at least one part of a drive device which can be coupled to the conveying device (5) of the applicator (1) in order to convey the radioactive substance into the application device (9) of the applicator (1), and a screening device (31, 33) for radioactive radiation so as to protect the hand holding the handle.

2. System according to claim 1, **characterized in that** the container (3) comprises at least a part (11) of a mixing device.

3. System according to claim 1 or 2, **characterized in that** the receiver device is formed by a clamping device (27), with which the applicator (1) can be held in a releasable manner.

4. System according to any one of claims 1 to 3, **characterized in that** the drive device comprises a thrust mechanism (45), with which a thrust force can be exerted onto the conveying device (5) of the applicator (1).

5. System according to claim 4, **characterized in that** it comprises a hand-held device (39), separated from the handle, on which the thrust mechanism (43) is arranged, and a guide wire (34), arranged at the handle (23) and capable of being coupled to the conveying device (5) of the applicator (1), which is connected to the thrust mechanism (43).

6. System according to claim 5, **characterized in that** the handle (23) exhibits an extended shape, wherein at one end the receiver device is arranged, and the handle comprises a channel (38) for receiving the guide wire (34), wherein the channel (38) traverses through the handle (23) in its longitudinal direction as far as the receiver device.

7. System according to any one of claims 1 to 6, **characterized in that** a part of the receiver device comprises a screening element (31), with which a part of an applicator (1) accommodated in the receiver device is covered.

8. System according to any one of claims 1 to 7, with a transport container (45) for holding one or more applicators (1).

9. System according to claim 8, **characterized in that** the transport container (45) comprises a part (48) of a magnetic mixing device, which can be coupled by means of a magnetic coupling to a further part (11) of the mixing device, arranged in the applicator (1).

10. System according to claim 8 or 9, **characterized in that** it comprises a frame, arranged in a movable manner on which are the transport container (45) and a holding element (52) for the handle (23), such that the handle (23) arranged in the holding device (52) can be moved to the transport container (45) for the removal of an applicator (1) being kept there.

## Revendications

1. Système comprenant un applicateur (1) pour l'application d'une substance radioactive sur un tissu, l'applicateur (1) comprenant un récipient tubulaire (3) pour le logement de la substance radioactive et un dispositif d'application (9), pouvant être relié avec une extrémité du récipient tubulaire (3), avec lequel la substance radioactive peut être appliquée sur le tissu, le dispositif d'application (9) comprenant une conduite (19), qui peut être introduite dans le récipient (3) pour l'établissement d'une liaison, afin d'introduire, après l'établissement de la liaison, la substance radioactive dans le dispositif d'application (9), et un pinceau, une spatule, une éponge, un petit tube ou une aiguille pour l'application de la substance radioactive sur le tissu biologique et le récipient tubulaire (3) comprend un poinçon ou un piston, en tant que partie d'un dispositif de convoyage (5) avec lequel la substance radioactive peut être introduite du récipient (3) dans le dispositif d'application (9) et l'applicateur (1) présente un état de stockage, dans lequel le récipient (3) et le dispositif d'application (9) sont fixés l'un à l'autre, une introduction de la substance radioactive du récipient (3) vers le dispositif d'application (9) étant empêchée par une fermeture (15) disposée entre le dispositif d'application (9) le récipient (3), le système comprenant une poignée (23) avec un dispositif de logement pour l'applicateur (1), au moins une partie d'un dispositif d'entraînement, pouvant être couplé avec le dispositif de convoyage (5) de l'applicateur (1), pour l'introduction de la substance radioactive dans le dispositif d'application (9) de l'applicateur (1) et d'un dispositif de blindage (31, 33) contre le rayonnement radioactif pour la protection de la main tenant la poignée.

2. Système selon la revendication 1, **caractérisé en ce que** le récipient (3) comprend au moins une partie (11) d'un dispositif de mélange.

3. Système selon la revendication 1 ou 2, **caractérisé en ce que** le dispositif de logement est constitué d'un dispositif de serrage (27) avec lequel l'applicateur (1) peut être maintenu de manière amovible.

4. Système selon l'une des revendications 1 à 3, **caractérisé en ce que** le dispositif d'entraînement comprend un mécanisme d'avance (43) avec lequel une force de poussée peut être exercée sur le dispositif de convoyage (5) de l'applicateur (1).

5. Système selon la revendication 4, **caractérisé en ce qu'**il comprend un appareil portatif (39) séparé de la poignée, sur lequel est disposé le mécanisme d'avance (43), et un fil de guidage (34), disposé sur la poignée (23) et pouvant être couplé avec le dispositif de convoyage (5) de l'applicateur (1), qui est relié avec le mécanisme d'avance (43).

6. Système selon la revendication 5, **caractérisé en ce que** la poignée (23) présente une forme allongée, moyennant quoi le dispositif de logement est disposé à une extrémité et la poignée comprend un canal (38) pour le logement du fil de guidage (34), le canal (38) traversant la poignée (23) dans sa direction longitudinale jusqu'au dispositif de logement.

7. Système selon l'une des revendications 1 à 6, **caractérisé en ce qu'**une partie du dispositif de logement comprend un blindage (31) avec lequel une partie d'un applicateur (1) logé dans le dispositif de logement est recouvert.

8. Système selon l'une des revendications 1 à 7, avec un récipient de transport (45) pour la conservation d'un ou plusieurs applicateurs (1).

9. Système selon la revendication 8, **caractérisé en ce que** le récipient de transport (45) comprend une partie (48) d'un dispositif de mélange magnétique qui peut être couplé par couplage magnétique avec une autre partie (11), disposée dans l'applicateur (1), du dispositif de mélange.

10. Système selon la revendication 8 ou 9, **caractérisé en ce qu'**il comprend un châssis que lequel le récipient de transport (45) et un support (52) pour la poignée (23) sont disposés de manière mobile, de façon à ce que la poignée (23) disposée dans le support (52) puisse être déplacée vers le récipient de transport (45) pour le retrait d'un applicateur (1) qui y est conservé.
